# EUROPEAN PATENT SPECIFICATION

(11) **EP 3 200 918 B1**
(45) Date of publication and mention of the grant of the patent: **05.05.2021**
(21) Application number: 15782007.7
(22) Date of filing: 30.09.2015
(51) Int. Cl.: B01L 7/00, B01L 3/00

(54) **EASY TO USE LIQUID TESTING DEVICES**
BENUTZERFREUNDLICH FLÜSSIGKEITSTESTVORRICHTUNGEN
DISPOSITIFS D'ANALYSE DE LIQUIDE FACILES À UTILISER

(30) Priority: 30.09.2014 GB 201417271
(43) Date of publication of application: 09.08.2017
(73) Proprietor: Brightwater Diagnostics Limited, Bath Bath and North East Somerset BA2 6BZ (GB)
(72) Inventor: GUNDRY, Stephen Walter, Bath BA2 6BZ (GB); GUNDRY, Benedict Foster, Bath BA2 6BZ (GB)
(74) Representative: Abel & Imray
(86) International application number: PCT/GB2015/052847
(87) International publication number: WO 2016/051167

(56) References cited:
- US-A- 4 976 923
- US-A- 5 119 830
- US-A- 5 429 804
- US-A- 5 501 837
- US-A- 5 595 187
- US-A1- 2012 106 811
- US-A1- 2013 089 474
- US-B1- 6 168 758

## Description

### Introduction

There is a need for improved methods, devices and apparatus for the testing of water and other liquids for microbiological and/or chemical quality. For example, for the testing of drinking water supplies for bacterial contaminants, the testing of water used in agriculture or food production/processing, the testing of environmental waters such as ballast water of ships or industrial discharge waters, or the testing of a urine sample for bacteria or for chemicals such as drug metabolites.

It is generally desirable for testing devices to be simple in operation so that the risk of user error is mitigated and so the need for user training is reduced. Such considerations may make the device more suitable for field use away from specialised laboratory staff. Such considerations may be also relevant if the test is to be used in a developing country where there is often a pressing need for testing liquids, for example a need to test water supplies for contamination. In general it is desirable for testing devices to be relatively low cost to manufacture. For applications in the developing world low cost may be a necessary prerequisite for the adoption of a testing regime. In the developed world, low cost may allow a test device to be a single use disposable device in order to prevent cross-contamination between samples. Generally, a test device which is simple, low cost and easy to use will usually result in more frequent testing being carried out to the benefit of efforts to monitor the quality of the liquid.

For many test applications, there is an advantage in collecting an initial quantity of liquid in a primary chamber of a device and then distributing at least some of that liquid into separate aliquots in multiple secondary regions, for example secondary chambers, of the device. Each secondary region may then be used to carry out a different test (for example a test for one of several chemical contaminants). Alternatively, when the device is used to detect microbial contamination of a liquid, the same test may be carried out in multiple secondary regions of the device and the proportion of multiple secondary regions testing positive for the microbial contaminant, combined with a knowledge of the ratio between the initial volume of liquid taken into the primary chamber and the volume of liquid aliquots taken into the secondary regions can be used to provide an approximate quantification of contamination level by use of the most probable number count methodology (the method of Poisson zeroes) whereby quantitative or semi-quantitative data is generated from incidence data.

In many applications there is an additional requirement that the collecting of an initial quantity of liquid involves the collection of a specific volume of liquid, for example a specific volume as defined or recommended by an appropriate regulatory or technical standard. There may be advantages if the correct initial volume can be collected directly into the primary chamber without requiring intermediate transfer for example decanting, pipetting or measuring steps.

It is a general requirement of a test device employing multiple regions that the regions are functionally isolated from each other such that cross-contamination between them is minimised to an extent such as the test results are not materially affected.

### Background

WO 2012/117244 discloses advice for receiving a fluid sample into an internal space and then distributing aliquots of that fluid sample into multiple chambers, wherein the fluid in each chamber is sealed to prevent cross-contamination.

The present invention is based in part on a realisation that whilst the secondary regions need to be isolated from the primary chamber to the extent that material contamination from the primary chamber into the secondary regions is avoided, it does not necessarily prevent proper functioning of the device if there is a risk of some contamination from one or more of the secondary regions into the primary chamber. Such a realization is especially relevant where the test carried out is a most probable number microbial test such that the volume of the test liquid in the primary chamber is many times larger than the volumes of the liquid in the secondary regions or chambers.

Some embodiments of the device of the present invention use absorbent material to retain liquid in the secondary regions as opposed to mechanically complex sealing mechanisms. Assay devices using absorbent material are known. Typically an absorbent material is used to wick the sample liquid from a collection region to an assay region by lateral flow. Such devices include that described in US 2013/0280698. However, the present invention is not a lateral flow device, rather absorbent material is used to retain the sample liquid in an secondary region, which is held in functional isolation from other secondary regions, and wherein the assay takes place *in situ* in at least some of the secondary regions, and in many embodiments in all of the secondary regions, rather than transport across a secondary region to a tertiary region in which the assay takes place.

US 5,501,837 discloses a medical test device having a lower cup and an upper lid which holds reagent strips. A similar device is disclosed in US 5,595,187, US 5,429,804, US 4,976,923, US 5,119,830, US 2013/0089474, US 2012/0106811 and US 6,168,758.

### Summary of Invention

According to a first aspect of the invention there is provided a test device for carrying out a most probable number microbial assay comprising a primary chamber (10) to receive an initial liquid sample (40) and a multiplicity of secondary chambers (21, 22, 23, 24, 25, 26) arranged to each receive an aliquot of the initial liquid sample from the primary chamber, wherein the test device is movable in use between two positions, a first position and a second position; characterised in that in the first position the secondary chambers (21, 22, 23, 24, 25, 26 are situated below the primary chamber (10) such that an aliquot of the initial liquid sample (40) flows under gravity from the primary chamber (10) to each of the secondary chambers (21, 22, 23, 24, 25, 26); and that in the second position the secondary chambers (21, 22, 23, 24, 25, 26) are located above the primary chamber (10) such that the liquid in the primary chamber (10) is not able to flow under gravity from the primary chamber (10) to each of the secondary chambers (21, 22, 23, 24, 25, 26); the aliquots of liquid sample which previously flowed under gravity from the primary chamber (10) to the secondary chambers (21, 22, 23, 24, 25, 26) when the device was in the first position being retained in the secondary chambers (21, 22, 23, 24, 25, 26) against the force of gravity and wherein the device is arranged so that an assay may be carried out in situ in at least some of the secondary chambers (21, 22, 23, 24, 25, 26), characterised in that the test device (1) is provided with a drip management system (80, 81, 82, 83, 84), wherein the test device is arranged so that, when the device is in the second position, any drips and surface flows of liquid flowing out of the secondary chambers (21, 22, 23, 24, 25, 26) are encouraged to flow or drip downwards into, or at least towards the primary chamber (10) in order to move them away from the regions of the device comprising the secondary chambers (21, 22, 23, 24, 25, 26) where they might contribute to contamination occurring between secondary chambers (21, 22, 23, 24, 25, 26) and wherein the volume of the primary chamber (10) is at least an order of magnitude greater than the volume of each of the secondary chambers (21, 22, 23, 24, 25, 26).

According to a second aspect of the invention there is provided a method of carrying out a most probable number assay for a microbial organism, comprising the steps of:
A, introducing a liquid sample (40) into a primary chamber (10) of a test device (1),
B, positioning the test device (1) such that an aliquot of the liquid sample from the primary chamber (10) flows into each of a multiplicity of secondary chambers (21, 22, 23, 24, 25, 26),
C, inverting the test device such that the aliquots of liquid sample in the secondary chambers (21, 22, 23, 24, 25, 26) are no longer in contact with the liquid sample remaining in the primary chamber (10),
D, allowing an assay reaction to take place in situ in the secondary chambers (21, 22, 23, 24, 25, 26) and, optionally the primary chamber (10),
E, reading the results of the assay reaction characterised in that the test device (1) is provided with a drip management system (80, 81, 82, 83, 84), wherein the test device is arranged so that, when the device is in the second position, any drips and surface flows of liquid flowing out of the secondary chambers (21, 22, 23, 24, 25, 26) are encouraged to flow or drip downwards into, or at least towards the primary chamber (10) in order to move them away from the regions of the device comprising the secondary chambers (21, 22, 23, 24, 25, 26) where they might contribute to contamination occurring between secondary chambers (21, 22, 23, 24, 25, 26) and wherein the volume of the primary chamber (10) is at least an order of magnitude greater than the volume of each of the secondary chambers (21, 22, 23, 24, 25, 26).

### Description of the drawings

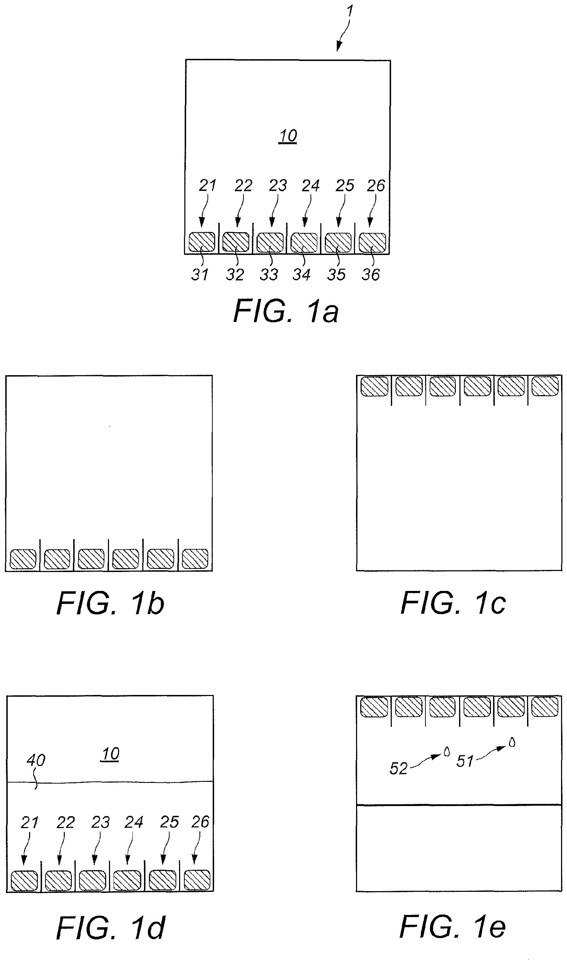

An embodiment of the invention in its first aspect is described below with reference to Figure 1, which is a schematic figure presented to show the principle of the invention. According to the invention there is provided a test device (Figure 1a, 1) comprising a primary chamber (10) and a multiplicity of secondary chambers (Figure 1a, 21, 22, 23, 24, 25, 26). The primary chambers each contain an individual portion of liquid-absorbent material (Figure 1a, 31, 32, 33, 34, 35, 36). The device is arranged to be movable between a first position and a second position. Figure 1b shows the device in the first position where the secondary chambers are situated below the primary chamber. Figure 1c shows the device in the second position where the secondary chambers are situated above the primary chamber. Figure 1d shows the device in use. An initial liquid sample (40) is received (by means not illustrated in Figure 1d) into primary chamber (10) and because secondary chambers (21, 22, 23, 24, 25, 26) are situated below the primary chamber (10) an aliquot of liquid sample flows into each of the secondary chambers. The device can then be moved from the first position to the second position as shown in Figure 1e. In the second position the secondary chambers are above the primary chamber. The aliquots of liquid sample are retained at least partially in the secondary chambers by being absorbed onto or into absorbent material in those chambers. When in the second position it can be seen that the content of each of the secondary chambers is functionally isolated from each of the other secondary chambers. As a result, each of the secondary chambers can be used for an independent assay without cross-contamination between them. There is also functional isolation between the primary chamber and the secondary chambers when the device is in the second position. At least initially, some liquid (51, 52) may drip from one or more secondary chambers into the liquid (40) in the primary chamber and potentially contaminate it. However, the present invention is based, in part, on the realisation that this is of no consequence in a most probable number microbial assay, because the volume of the liquid in the primary chamber is significantly larger than the liquid volume in the secondary chambers, microbial contamination from a secondary chamber to the primary chamber does not matter, because if one of the secondary chambers is positive for a viable microbe, probabilistically the primary chamber is likely to be positive for that microbe anyway, even before any such contamination takes place. There is also the likelihood that, because drips from the secondary chamber to the primary chamber will most likely only occur shortly after the device is placed in the second position, any microbes will still be in the lag phase of growth where there has been no increase in microbial populations in any chamber thus the secondary chambers will have the same concentration of microbial cells as the primary chamber such that any transfer by a drip from one chamber to another will not result in a significant change in microbial cell numbers in any chamber.

The device is provided with a "drip management system" wherein any drips of liquid from any secondary chamber are encouraged to fall into the primary chamber to prevent their contaminating other secondary chambers.

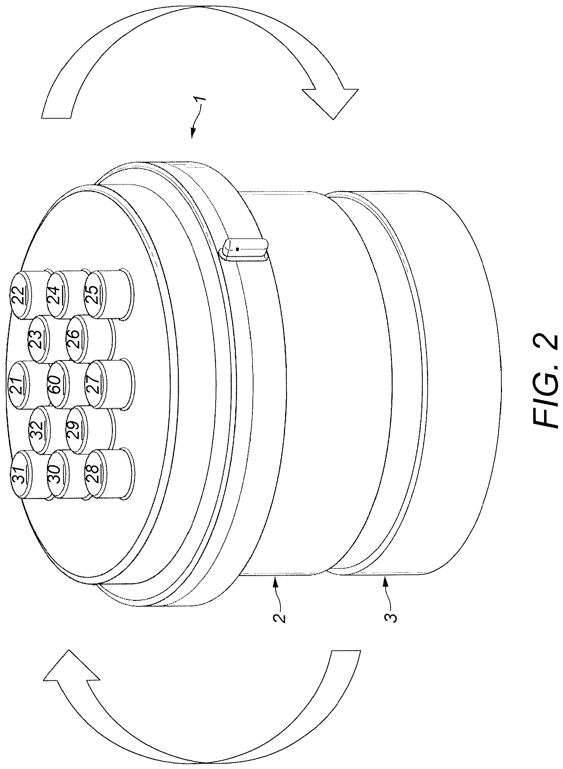

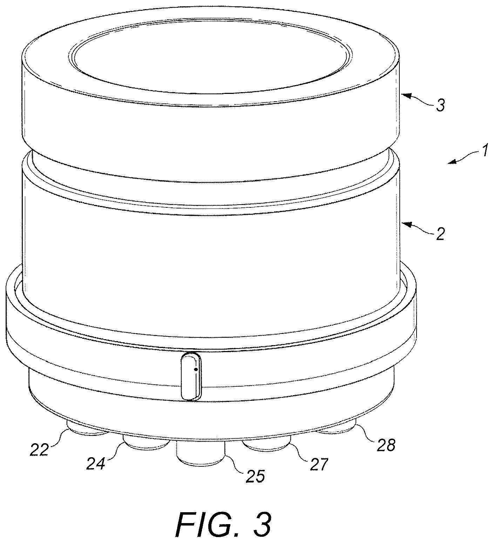

The device may conveniently be provided in two screw-together parts such that the primary chamber splits into a base portion and a lid portion and the secondary chambers are provided on one of those portions. This allows the chamber to be opened to receive an initial liquid sample and then be screwed together to seal the liquid sample inside. Figures 2 and 3 show a possible configuration wherein device (1) consists of a base portion (2) and a lid (3). It can be seen that the device is arranged so that it can sit on its flat base when in a first position and so that it can sit on its flat lid when in the second position. The secondary chambers (21, 22, 23, 24, 25, 26, 27, 28, 29, 30, 31) in this configuration are mounted on the base section.

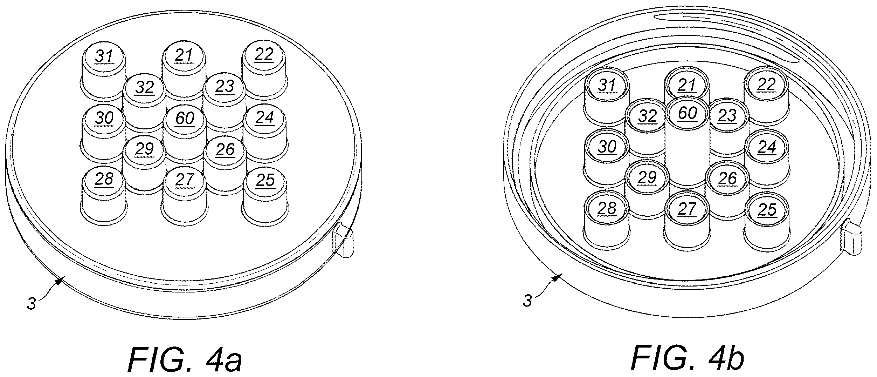

Figures 4, 5, 6, 7, 8, and 9 show an alternative configuration wherein the secondary chambers are mounted on the lid portion. Such an arrangement has the advantage that the base portion (2) may be a standard sample collecting vessel (for example a collecting jar, sample container, bottle or tub), which may be disposable or re-usable. This may reduce the cost of the device by allowing for use of standard components. It also permits the sample to be initially collected in a standard collecting vessel with a standard lid and for the standard lid to be replaced by a 'diagnostic' lid, thereby forming the device of the invention, at a later time. It also allows the option of using more than one diagnostic lid with the standard collecting vessel of initial liquid sample, either as alternatives or sequentially. Having the secondary chambers in the lid also allows the sample to be given time to 'settle' in the base portion before the lid is applied; this allows time for any foam or silt or other material to come out of the liquid and reduces the risk of components of the diagnostic lid clogging. The use of a diagnostic lid fitted to a standard sample collecting vessel also facilitates, should the method of use require, the mixing of the sample with a reagent because the sample and reagent may be mixed (for example by shaking) in a standard collecting vessel with a standard lid prior to the standard lid being removed and replaced by the diagnostic lid. Mixing with a standard lid in place rather than a diagnostic lid avoids the risk of secondary chambers becoming clogged with undissolved reagent and/or being exposed to the incorrect reagent concentration.

Figures 3 to 9 show some details of the secondary chambers and absorbent material. It will be understood that those features are equally applicable to the configuration shown in Figures 1 and 2.

As can be seen from Figure 4a (and also Figure 2) the secondary chambers each present a flat surface on the top of the device when it is in the second position. If the secondary chamber wall is made of transparent or translucent material this allows the diagnostic signal produced by the test taking place in the secondary chambers to be read out either as a colour change or, with the aid of an appropriate apparatus, a fluorescent or other signal. It can also be seen that there is an additional feature present which is optional and not shown in Figure 1. That feature is referred to as the 'snorkel' (60) and is provided to provide a convenient read-out of the result of any test assay performed in the primary chamber. Whilst such a result could be read out directly from the primary chamber, for example through the device side wall, by bringing the result up to the same plane as the secondary chambers, it may be conveniently read at the same time and/or with the same apparatus.

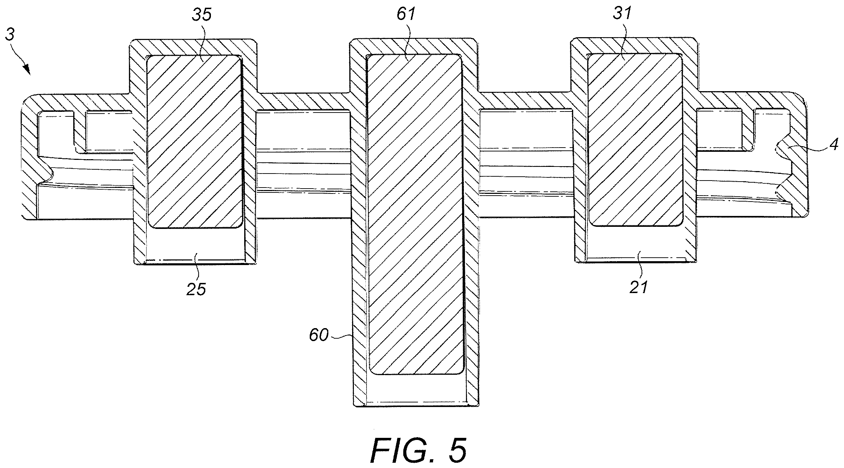

As can be seen from Figure 5, the lid portion (3) is provided with a screw thread (4) so that it may be conveniently fixed to the base portion. The secondary chambers are at least partially filled by absorbent material (31, 35). Absorbent material (61) is also provided in the snorkel (60).

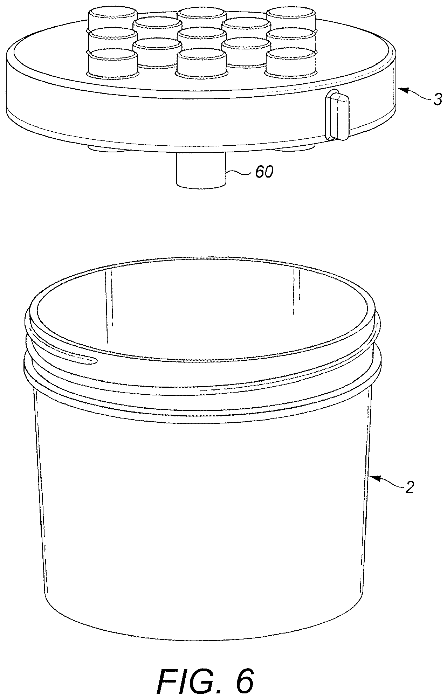

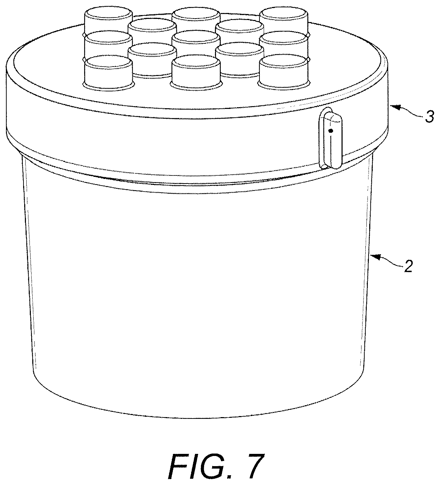

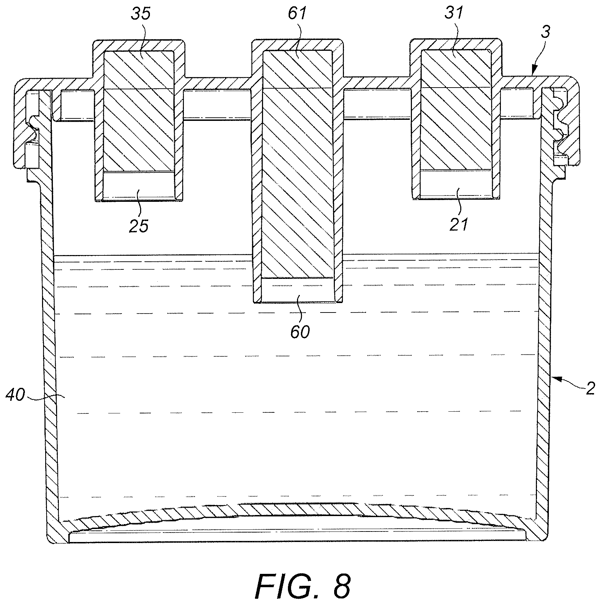

Figure 6 shows the lid portion above the base portion prior to being attached via the screw thread. As can be seen the snorkel (60) protrudes further downwards such that when the lid is attached (Figure 7), the snorkel is in contact with the sample (Figure 8, 40) in the primary chamber when the device is in the second position and the secondary chambers are not in contact with the sample in the primary chamber.

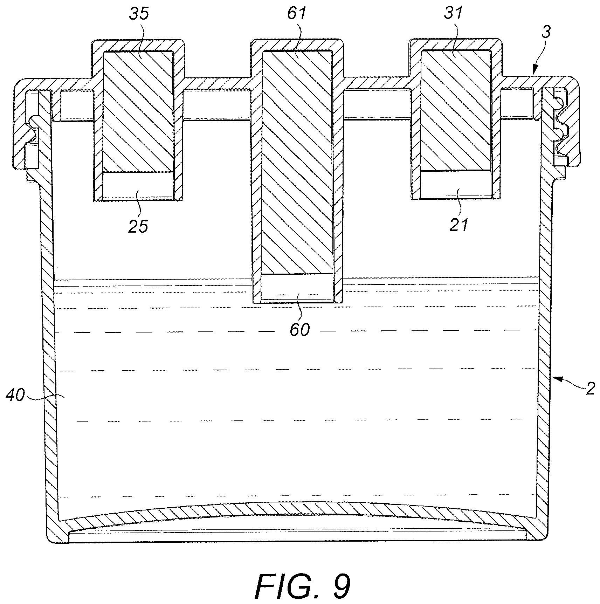

Figure 9 shows that even when the device has been placed into the primary position and some of the sample has been absorbed into the absorbent material in the secondary chambers before the device is placed in the second position and consequently the liquid level in the primary chamber has dropped (cf. the level in Figure 8 versus Figure 9), that the snorkel (60) is still in contact with the liquid (40) in the primary chamber.

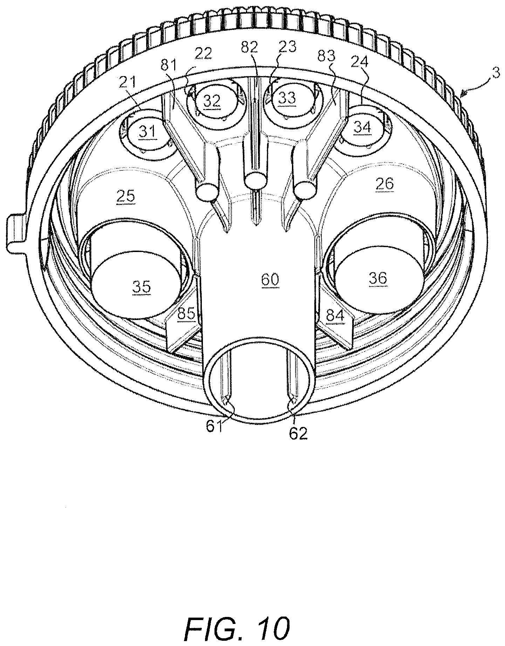

Figure 10 shows an alternative lid portion (3). This alternative differs from the lids shown in the previous drawings in a number of aspects. In common with the embodiment shown in Figure 5, this embodiment has a central snorkel (60) which extends further into the primary chamber of the device. The snorkel (60) in use contains absorbent material, although the view shown in Figure 10 shows the snorkel (60) and optional ridges 61 and 62 internal to the snorkel which are provided to enhance the retention of the absorbent material. Similar optional ridges may be provided in the secondary chambers also. In the embodiment illustrated in figure 10. The absorbent material in the snorkel is not visible because it fills the upper part of the snorkel only. In other embodiments it may fill all of the snorkel or even extend beyond the open end of the snorkel. Further advantages of the ridges are discussed below. The lid also shows a number of secondary chambers (31, 32, 33, 34, 35 and 36) of two different sizes. As can be seen, the secondary chambers are filled with absorbent material which in the case of chambers 25 and 26 extends beyond the chambers. As can be seen in chambers 25 and 26, the chamber side wall does not extend towards the primary chamber to the same extent along all of its circumference. This means that any excess liquid on the secondary chamber wall, for example on the outside of the secondary chamber wall will, when the device is in the second position, tend to flow to and along the edge of the secondary chamber wall and towards the snorkel. The provision of a moulded piece between the secondary chamber and the snorkel (piece 70) will allow the liquid to flow down the snorkel wall. By such an arrangement, this liquid is drawn away from regions of the device between the secondary chambers where it may undesirably contribute to a liquid bridge between secondary chambers which during a microbial growth phase could facilitate cross contamination between the secondary chambers. A similar arrangement is provided for the smaller secondary chambers (21, 22, 23, 24) and so for the inner surface of the lid such that, rather than being flat, it slopes towards the snorkel to encourage drainage of liquid from the regions between the secondary chambers.

The device is also provided with further contamination reducing means in the form of "cattle stall" barriers 80, 81, 82, 83, 84. These are walls provided between the secondary chambers arranged so that any liquid on them tends to drain to their lower edge. The lower edge is preferably sloping so that liquid on it tends to flow towards the snorkel or the ejector pins pads/bosses 90, 91, 92, where it is unlikely to cause problematic bridging between secondary chambers and, therefore, cross-contamination. Arrows in Figure 10 show the direction in which liquid will tend to flow when the device is in the first position.

### Detailed Description of Invention

According to a first aspect of the invention there is provided a test device for carrying out a most probable number microbial assay comprising a primary chamber (10) to receive an initial liquid sample (40) and a multiplicity of secondary chambers (21, 22, 23, 24, 25, 26) arranged to each receive an aliquot of the initial liquid sample from the primary chamber, wherein the test device is movable in use between two positions, a first position and a second position; characterised in that in the first position the secondary chambers (21, 22, 23, 24, 25, 26 are situated below the primary chamber (10) such that an aliquot of the initial liquid sample (40) flows under gravity from the primary chamber (10) to each of the secondary chambers (21, 22, 23, 24, 25, 26); and that in the second position the secondary chambers (21, 22, 23, 24, 25, 26) are located above the primary chamber (10) such that the liquid in the primary chamber (10) is not able to flow under gravity from the primary chamber (10) to each of the secondary chambers (21, 22, 23, 24, 25, 26); the aliquots of liquid sample which previously flowed under gravity from the primary chamber (10) to the secondary chambers (21, 22, 23, 24, 25, 26) when the device was in the first position being retained in the secondary chambers (21, 22, 23, 24, 25, 26) against the force of gravity and wherein the device is arranged so that an assay may be carried out in situ in at least some of the secondary chambers (21, 22, 23, 24, 25, 26), characterised in that the test device (1) is provided with a drip management system (80, 81, 82, 83, 84), wherein the test device is arranged so that, when the device is in the second position, any drips and surface flows of liquid flowing out of the secondary chambers (21, 22, 23, 24, 25, 26) are encouraged to flow or drip downwards into, or at least towards the primary chamber (10) in order to move them away from the regions of the device comprising the secondary chambers (21, 22, 23, 24, 25, 26) where they might contribute to contamination occurring between secondary chambers (21, 22, 23, 24, 25, 26) and wherein the volume of the primary chamber (10) is at least an order of magnitude greater than the volume of each of the secondary chambers (21, 22, 23, 24, 25, 26).

The condition that the assay be carried out in at least some of the secondary chambers means that liquid retained in the secondary chamber is subjected to an assay wherein a read-out, be that chromogenic, fluorescent or electrical or otherwise is obtained from the liquid retained in the secondary chamber. This is in contrast to arrangements wherein liquid is transported by a wicking action from an initial secondary chamber to a separate assay region.

Preferably, the device is arranged such that the sample liquid filled device need only be placed in the first position for a few seconds (for example up to 2, 4, 10, 20, 40, 60, 80, 100 or 120 seconds) before being returned to the first position.

### First Position and Second position

The test device is preferably arranged so that it may be moved between the first and second positions by being inverted. For example, the device may have two ends, such that it is in the first position when placed on one end and in the second position when placed on the other end. To facilitate this and to provide the device in an advantageous screw-together configuration, the device is preferably generally cylindrical in overall shape.

### Types of Secondary Chambers

A requirement of the secondary chamber is that it is able to retain liquid against the force of gravity when the device is in the second position. There are alternative ways in which this may be achieved. According to some preferred embodiments the liquid may be retained in or on absorbent material which may either be contained in a secondary chamber containing (either fully or partly) absorbent material. Alternatively, the secondary chambers may incorporate a pressure-operated microvalve such that liquid is retained in the secondary chamber.

### Absorbent Material

The secondary chambers may comprise absorbent material either contained in a chamber or attached to a surface of the device. In some embodiments the secondary chambers may combine small separate regions of absorbent matter which has been 3D printed onto an inner surface of the device. Alternatively small absorbent regions may be moulded integral to the wall of the device or attached by other microfabrication techniques. The regions may be arranged so as to form a 2D barcode pattern, preferably viewable through a transparent wall of the device.

The assay takes place in the secondary chamber. In embodiments wherein the secondary chamber contains absorbent material it is preferred that the assay takes place in and on the surface of the absorbent material throughout the secondary chamber. The absorbent material functions to retain an appropriate amount of the liquid in the whole of the secondary chamber rather than merely to transport some of the absorbed liquid out of the secondary chamber to a functionally separate assay region. It is preferred that the absorbent material is fully wetted when the device is placed in the first position and that that is achieved in a short time (for example up to 2, 4, 10, 20, 40, 60, 80, 100 or 120 seconds) during which the device is in the first position and the sample liquid is in the device.

As used herein the term "absorbent material" includes microstructures and liquid trapping and retaining surfaces which may be attached to or integral with the surface of the device (for example moulded into the walls of the secondary chamber. Examples of microstructures encompassed by the term "absorbent material" as used in the present invention include any microstructure, for example capillary structures, arranged to abstract, retain and/or contain a portion of liquid.

### Types of chambers

Typically the secondary chambers have walls which may optionally be integrally moulded with the rest of the device. In the embodiment in which they are at least partially filled with absorbent material the secondary chambers preferably have an open end which is towards the bottom of the chamber when in the second position and through which liquid may enter when the device is in the first position. Preferably, the secondary chambers are generally cylindrical, having two ends and a curved side wall.

Alternatively the secondary chambers may merely be dimples or depressions in the internal wall of the device.

In embodiments with absorbent material that material may optionally be adhered to a wall of the secondary chambers or alternatively, it may be wedged into the secondary chambers against the walls of the chambers. Alternatively the absorbent material may be integral to the walls of the secondary chamber, for example as microstructures printed onto, or moulded into the walls.

### Means of retaining liquid in secondary chambers

As discussed above liquid may be retained in secondary chambers by either a valve or absorbent material. It will also be understood that there may be embodiments wherein both valves and absorbent material are used together.

### Valves

According to some embodiments the aliquots of liquid are retained in the secondary chambers by the presence of fluid valves in the entrance of the secondary chambers. Such valves may be arranged so that when the device is in the first position, the valves open under the pressure of the liquid external to the secondary chambers, allowing liquid to be admitted. When the device is in the second position the pressure of the liquid in the secondary chambers in the opposite direction results in valve closure and retention of the liquid in the chamber. Suitable valves include those made by Minivalve International, Oldenzaal, The Netherlands.

### Absorbent material

In preferred embodiments the liquid is retained in the secondary chamber by means of liquid absorbent material as described herein.

### Configuration of test device

There are various possible configurations of the device which permits it to be moved between a first position and second position such that when the device is in the first position the primary chamber is above the secondary chambers and in functional communication and when the device is in the second position the secondary chambers are above the primary chamber and no longer in functional communication with the primary chamber. The simplest, and therefore preferred arrangement, is simply to provide the device with at least two flat external surfaces such that when placed on a level surface, such as a desk, bench or table top or incubator shelf, on the first flat exterior surface the device is stably in the first position and, when placed on the second flat exterior surface, the device is stably in the second position. The device can be simply moved by hand from the first to the second position. Alternatively, or optionally and additionally, the external shape of the device can be configured such that the device can be received into a supporting apparatus or stand which holds it stably in the first position and the second position. If the device is for use in an assay which requires incubation under defined conditions (for example at a defined temperature) the supporting apparatus may optionally incorporate means to maintain those conditions (for example heating or cooling means). The external surface of the device may optionally include handles, lugs, detents, written or symbolic instructions, and/or other surface features to facilitate movement between the first and second position either by hand or a robotic or other mechanical device and/or to allow the device to be held in the correct orientation in the supporting apparatus.

The device requires a means for the initial liquid sample to be received into the primary chamber. Various means are envisaged including syringe ports, but for most uses the simplest and therefore preferred means for the initial liquid sample to be received into the primary chamber is to provide the device in two or more parts such that the two parts, which may be referred to as a lid portion and a base portion, are able to be joined together to form the complete device. Preferably, the portions are joined by a conventional screw thread optionally incorporating a sealing means such as a gasket of resilient material or a deformable plastic inner rim in order to prevent leaks. In use, such a device will have the lid portion removed and the base portion will be filled with the initial liquid sample, for example by having the sample poured in or by scooping the sample up directly.

The base portion may be optionally marked with filling instructions, for example a fill line to which the device is to be filled in order to fill the device with the correct volume of liquid.

The combination of a device comprising two screw-together portions and the requirement for two external flat surfaces mean that the preferred overall shape of the device is generally cylindrical as shown in the figures.

In some embodiments the secondary chambers may be provided as part of the lid portion; in other embodiments the secondary chambers may be provided as part of the base portion. The relative merits of these alternative configurations are discussed elsewhere in this specification. It will be appreciated that, when the secondary chambers are provided as part of the lid portion (the so-called "diagnostic lid" configuration) the device may be provided with a standard lid also, for use in closing the base portion prior to fitting the diagnostic lid, for example for closing the base portion during transport of the device, for example between a sample collection site and a sample processing site, for example between a field site and a local work station of office some distance from the field. Having a base portion which is compatible with a standard lid is especially advantageous when the initially collected sample needs to be shaken or inverted before the diagnostic lid is fitted in order to mix and/or solubilise an assay reagent.

Preferably, the volume of the primary chamber is significantly greater, for example at least an order of magnitude greater, than the volume of each of the secondary chambers. For example, the primary chamber may have a volume of at least 100 ml so that it can receive (to optional fill line) a volume of 100 ml of liquid sample. The volumes of each of the secondary chambers may be 1 or 2 ml, or 0.2 ml or 0.5 ml. In some embodiments where the secondary chambers are small structures the volume of liquid retained in them may be many times smaller, for example 1, 2, 4, 5, 10 or 100 microliters. In some embodiments all secondary chambers will be of the same volume. In all embodiments, the secondary chambers will each be of significantly lower volume than the primary chamber, for example at least 10, at least 20, at least 40, at least 50, at least 80, at least 100, at least 200, at least 500, at least 1000, at least 5000 times lower volume.

According to all embodiments there are at least 2 secondary chambers. There may be 3, 4, 5, 6, 7, 8, 9, 10, 11, 12, 13, 14, 15, or more secondary chambers, for example more than 3, more than 5, more than 8, more than 10, more than 12 secondary chambers. Alternatively, there may be at least 20, 40, 60, 80, 100 or 200 secondary chambers.

In certain embodiments the device has multiple secondary chambers of a first size and then a number of further secondary chambers of a second, larger size in order to provide greater sensitivity at the lower end of the detection range when used in a most probable number microbial assay. For example the device may include 8 secondary chambers of 0.2 ml volume and 2 secondary chambers of 2 ml volume.

In certain embodiments, the secondary chambers may be arranged such that they form a pattern either on their own or in combination with other features of the device such that an image of the chambers taken with a camera may be analysed by image processing software so that individual secondary chambers are identified. In some embodiments the secondary chambers may be arranged and/or shaped to become part of a 2D barcode (for example a Quick Response code to ISO/IEC 18004:2006) wherein a colour change reaction in the secondary chambers results in a pixel of the 2D barcode changing its binary value. In such an arrangement it is only necessary that some of the pixels correspond to the result of a test reaction. Other pixels may be provided to encode a serial number, a test type identifier or positioning and orientation information. Those 'fixed pixels' may be provided by printing onto the device surface or by any other means. In such embodiments it may be desirable for the secondary chambers to be provided at relatively high density. It may be preferable to achieve that by providing secondary chambers containing absorbent material which are minimal dimples or depressions or as secondary chambers formed with microstructures during the manufacture of the rest of the device, or, for example, co-moulded with the rest of the device.

Examples of 2D barcodes which may be used include those meeting the SPARQCode standard, the ShotCode standard, the QRCode standard, the QCode standard, the PDF417 standard, the NexCode standard, the MaxiCode standard, the EZCode standard, or the Aztec Code standard. Alternatively, the 2D barcode may be in accordance with a specially developed standard but, in order to qualify as a 2D barcode, at least some pixels need to be used to provide positioning and orientation information. The use of a 2D barcode may be advantageous because it can be read with a readily available digital camera, for example a camera provided as part of a cellular mobile telephone. With appropriate software, a cellular mobile phone can be used to capture the image, derive data from it, report that data and optionally transmit it by wireless telephony, without the need for specialist equipment.

It will be appreciated that, in all aspects, the device of the invention preferably operates with no moving parts such that the device itself is moved between the first and second positions, but the device itself has no moving parts. In particular, the functional separation of the secondary chambers from each other and the primary chamber does not require the user to do anything other than invert the device. User operation of sliders, clips, screw down lids, plugs and other moveable parts is not required in order to place the secondary chambers in functional isolation.

### Test 'snorkel'

An optional feature in accordance with embodiments of the invention is the provision of a 'snorkel'. Preferably, such a feature is provided on the portion of the device (lid or base portion) on which the secondary chambers are installed. It is arranged such that it is in contact with the liquid in the primary chamber when the device is in both the first position and the second position and both before and after aliquots of the initial liquid sample have been taken into the secondary chambers.

The simplest way of providing this functionality is to construct the 'snorkel' so that it protrudes into the primary chamber further than do the secondary chambers such that the end of it remains submerged in the liquid sample in the primary chamber even when the device is in the second position.

A purpose of the 'snorkel' is so that the results of whatever test assay may be carried out on the sample in the primary chamber are visible (either directly to the eye or to an instrument) next to the results of the test assays carried out on the sample aliquots in the secondary chambers. By placing all of the results together, preferably on a single side of the test device, all of the results can be read together. The liquid retained in the snorkel may be considered as an integral part of the liquid in the primary chamber such that when the device is used for a microbiological assay any microorganisms incubated in the primary chamber will grow equally in the snorkel as in the rest of the primary chamber.

Preferably, there is a single snorkel. However, in certain embodiments it may be preferred to provide multiple snorkels (for example 2, 3 or 4).

The snorkel may be wholly or partially filled with absorbent material

### Drip Management System

The device is provided with a drip management system. This comprises features of the device arranged so that when the device is in the second position, any drips or surface flows of liquid flowing out of the secondary chambers are encouraged to flow or drip downwards into, or at least towards the primary chamber in order to move them away from the region of the device comprising the secondary chambers where they might contribute to contamination occurring between secondary chambers. The most straightforward drip management system may be to provide secondary chambers wherein the side walls of the chambers are configured to be higher at one point of the wall than other parts of the wall such that when the device is in the second position any liquid which is not retained in a secondary chamber, for example by the absorbent material, flows down the side wall of the secondary chamber (by surface-directed flow) and towards the highest region of the wall which, when the device is in the second position is the region of the wall which is lowest in the device. From that region of the wall, the liquid may form a drip and drip into the primary chamber or more preferably continue in its downward surface-directed flow generally towards the primary chamber along a surface or channel provided in the device. Preferably, the device is configured with a multiplicity of secondary chambers at one end of the device, the secondary chambers being arranged around a snorkel, and the secondary chambers comprising a side wall which extends generally in the direction of the primary chamber wherein the side wall has a region where the side wall extends further towards the primary chamber than the rest of the side wall so that surface flow of liquid along the side wall to this region is encouraged. Preferably, the device is provided with a "drip guide" region extending from the extended region of the side wall of the secondary chambers to the snorkel of the device so that liquid flowing down the side walls of the secondary chamber is directed by surface flow away from the secondary chambers towards the snorkel and optionally into the primary chamber.

### Materials for construction

The main body of the device may be constructed out of any suitable material such as glass or plastic material. For applications wherein the device is to be at least partially re-usable, glass may be a suitable and preferred material. In other embodiments the device is most preferably constructed out of a moulded plastics material, for example, polypropylene, polyethylene, polycarbonate, copolyester or polystyrene. If the device features a screw thread then that may optionally be provided with a resilient gasket made, for example, out of a rubber or silicone compound. Alternatively, a suitable seal may be provided without the use of a gasket or by the provision of a deformable rim internal to the screw thread.

Preferably, the material used in constructing the chamber is sufficiently transparent to enable a user to see whether the secondary chambers are full of liquid or whether the device is empty, and also to allow any test results to be read out as a colour change or other signal through an external wall of the device, either by eye or by an instrument (for example an instrument which detects a fluorescent or colour change signal in the visible or invisible portion of the electromagnetic spectrum). In certain embodiments, only the walls of the secondary chambers, or those parts of the secondary chamber adjacent to the external surface of the device have sufficient transparency. For certain applications, it is preferred that the device be constructed out of material that does not interfere with the assay signal. For example when the assay signal is a fluorescent or colour change signal, it is preferred that the material of construction does not auto-fluoresce. It is also preferred that the material allows excitation wavelength (for example UV) light to pass into the chambers and that it allows light at the assay emission wavelength(s) to pass out of the chamber for viewing or detection.

Preferably, the material of construction, at least in the regions requiring transparency, is made of a transparent thermoplastic, for example a polyolefin such as a cyclic olefin copolymer (COC), a cyclic olefin polymer (COP) or an isotactic polyolefin. Suitable compounds include Poly(4-methyl-1-pentene) (sold as TPX™ by Mitsui Chemicals), and polymers of 8,9,10-trinorborn-2-ene or 1,2,3,4,4a,5,8,8a-octahydro-1,4:5,8-dimethanonaphthalene with ethene (sold as TOPAS™, as APEL™, ZEONOR™ or ARTON™ by various manufacturers including Mitsui Chemicals).

In certain embodiments it may be advantageous for the device to be made of different materials in different regions, such that the regions requiring transparency are made of a material with suitable optical properties and the rest of the device is made of a cheaper material. Different materials may be welded together for example by ultrasonic welding. In certain embodiments it may be advantageous for some of the device to be black or a similarly dark colour in order that contrast between the regions transmitting an assay signal and the rest of the device to be maximised. Alternatively, it may be advantageous to mould the whole device from the same material but to provide advantageous contrast by applying paint, ink or an opaque plastic or paper sticker (for example in black) to the regions of the device surrounding those transmitting an assay signal.

Optionally, and when the device is arranged for use in testing water or an aqueous liquid, all or part of the interior surface of the chambers, and in particular the secondary chambers, are hydrophobic in order to reduce the risk of liquid films forming on the surface and forming a functional link between the chambers when the device is in the second position. Such hydrophobicity may be provided as an inherent property of the material from which the device is constructed. Alternatively, the surfaces may be coated with a hydrophobic material. A hydrophobic material may be provided over all of the internal surfaces of the device or alternatively, it may be provided over part of the internal surface, for example around the external surface of the secondary chambers only. Surfaces may be treated to make them more hydrophobic. For example, a plasma treatment with CF₄ plasma may be used. Alternatively, or additionally, the device may be constructed from a plastics material in which an additive such as "hydromaster" available from Addmaster Limited, Stafford, UK has been added in order to increase the plastic's hydrophobicity.

To reduce the risk of biofilms forming a functional link between the secondary chambers of the device when the device is in the second position, various optional features may be employed, including:
1. configuring the device to complete the assay before sufficient time has elapsed for problematic biofilms to form;
2. using hydrophobic material as described above;
3. incorporating bacteriostatic or bactericidal materials into the device.

Care must be taken that the use of bactericidal material does not inhibit assays that require bacterial growth. The invention includes coating the internal surfaces of the device with bactericidal or bacteriostatic materials such that those materials do not prevent bacterial growth in the lumen and absorbent material in the secondary chamber or alternatively providing bactericidal material in part of the internal surface of the device only so as to form a biological "fire break", for example by providing bactericidal rings fitted around the external surface of the secondary chambers only. Alternatively the biological "fire breaks" may be constructed from a material to which an antimicrobial additive has been added, for example a silver-based additive available from Addmaster Ltd.

### Contamination Management System

According to certain embodiments, the device is constructed with separating walls between the secondary chambers. These separating walls being dimensioned so that any liquid on them, which could potentially provide a functional link between the secondary chambers of the device, are channelled by surface flow towards the snorkel, or otherwise in the general direction of the primary chamber.

Alternatively or additionally, the device may be configured such that, when the device is in the second position, the surfaces between the secondary chambers are not horizontal, but rather slope (preferably towards a snorkel) to reduce the retention of surface water and encourage drainage of the surfaces.

### Internal Pressure considerations

In certain uses, reactions in the sample (either assay reactions or side reactions) may produce or consume gas and this may lead to a change in internal pressure of the device relative to ambient pressure. Internal pressure may also change as a result of heating the device. If this is expected to be a potential problem (and in some applications it will not be), the device may either be constructed sufficiently robustly to contain such anticipated changes in pressure or the device provided with one or more pressure equalisation means, for example in the walls of the primary chamber. These means preferably allow for the passage of gas but the containment of liquid and may for example be patches of Gore-Tex™ or other breathable fabric or film welded or otherwise attached to a portion of the wall of the primary chamber. Alternatively, a degassing valve or a breather valve similar to those used in the food industry to allow gas to escape from packaged coffee whilst preventing contamination of the product may be used.

### Fluorescence Enhancement

Polyvinyl alcohol (PVA) may be used in accordance with the invention to enhance a fluorescence signal from an assay. It may be especially suitable to enhance a fluorescent signal from a cleavable indicator such as MUG (4-Methylumbelliferyl beta-D-glucuronide), X-gluc (5-Bromo-4-chloro-3-indoxyl-beta-D-glucuronide cyclohexylammonium salt) or X-gal (5-Bromo-4-chloro-3-indoxyl-α-D-galactopyranoside) or similar and derivative compounds. There are various ways of providing this enhancement according to the invention, including providing solubilized PVA in the secondary chambers, or in solution, or by providing solid PVA in the secondary chambers, including providing it as part of the absorbent material according to the invention, or by providing a thin disc of cast solid PVA at the bottom of each secondary chamber (between the absorbent material and the end wall of the chamber).

In some embodiments, the PVA may be provided as part of, or alongside, the test reagent.

### Absorbent material

In some embodiments the secondary chambers and, if present, the snorkel are at least partially filled with an absorbent material sufficient to retain liquid in the secondary chamber and, if present, the snorkel, when the device is in the second position. For the avoidance of doubt, it is stated that the term 'absorbent material' encompasses 'super absorbent materials'. The absorbent material may comprise cotton wadding, cellulose fibres, polyester fibres, rayon fibres, polypropylene fibres, polyester fibres or polyurethane, or a mixture of any thereof or the absorbent material may comprise a polyvinyl alcohol (PVA) foam or other absorbent foam or sponge material.

The term 'absorbent material' includes material with capillary properties. The term 'absorbent material' also includes microstructures able to absorb or retain liquid, for example microstructures moulded into or adhered to the wall of a chamber.

In certain embodiments the absorbent material may incorporate assay reagents or retain assay reagents between the absorbent material and the walls of the secondary chamber. In certain embodiments the absorbent material may be provided with channels or grooves which form channels when pressed against a secondary chamber wall. So that air may be assisted in escaping from the material during absorption, thereby facilitating rapid and complete absorption.

In certain embodiments, the secondary chamber wall may be provided with ridges for that purpose and/or to provide a tighter interference fit of the absorbent material.

In many embodiments it is important that the correct volume of liquid is taken into each secondary chamber. A challenge in ensuring that this happens consistently is the formation of airlocks in the secondary chambers. These can be prevented by providing ridges or grooves in the inner chamber side wall or in the absorbent material itself to ensure a path for air to escape from the chamber as it fills with liquid. The efficacy of the device relies in a fast uptake of liquid and this is achieved by ensuring that there is good airflow from the base of the chamber out of the chamber. The absorbent material will also preferably be chosen to achieve fast uptake of liquid and may, in some embodiments, be pre-treated to assist in fast uptake, for example with a wetting agent.

In certain embodiments, the secondary chambers may be filled with absorbent material, which extends beyond the walls of the secondary chamber. In other embodiments the absorbent material may only partially fill the chamber.

### Liquids

The sample liquid is preferably an aqueous liquid. More preferably it is a water sample, for example a sample of sea water, river water, lake water, stream water, pond water, waste water, well water or industrial process water. Most preferably it is a sample of putative drinking water for humans or animals or a sample of water for putative use in agriculture or horticulture or food production/processing for example as a putative crop irrigation water or a sample of putative bathing water. The sample may be an environmental water for example a sample taken from a ballast tank of a ship. The sample may be of water, for example drinking water which has been held in storage for future use. In some embodiments the water may be an industrial water such as a cooling water or air-conditioning water which is recirculated in use and is at risk of microbial contamination (for example by *Legionella*). Alternatively, the sample may be a sample obtained from a swab, such as a surface or hand swab which has been washed into a known volume of liquid to produce a liquid sample. In alternative embodiments the sample may be a liquid medical sample, for example a sample of pus, plasma, serum, or urine. In alternative embodiments the sample may be a sample of a food product, for example a sample of fruit juice, milk, beer, wine, cider, yoghurt etc. The sample may have been directly collected or pre-processed before introduction into the device of the invention (for example to dilute the sample, to clarify the sample or to remove sediment, foam, cells or other debris or components that might interfere with the test assay).

### Test assays

Preferably, the device is configured to provide a most probable number microbial assay for coliform bacteria for example *E. coli* in a water sample. Such a device may optionally and additionally provide further water quality assays in addition to the most probable number assay for coliform bacteria in a water sample, for example chemical assays of cyanide, heavy metal, phosphate or nitrate contamination of the water sample.

### Test reagents

In order to carry out an assay in the device, assay reagents must first come into contact with the liquid sample. In some embodiments this may be done prior to the liquid sample being introduced into the device. Alternatively, the reagents may be added to the liquid sample in the primary chamber. Alternatively, the device may be provided with an amount of reagent already in the primary chamber (for example, loose in the chamber in a liquid or solid, for example powdered, form or coated onto the wall of the chamber). Alternatively, or additionally, the device may be provided with an amount of reagent in each of the secondary chambers (for example as a liquid pre-absorbed onto the absorbent material in the chambers, or as a coating on the wall of the chambers or as a solid form such as a powder, held in the chamber between the chamber wall and the absorbent material. Adding the reagent into the primary chamber has the advantage that it can be used in multiple assays in each of the secondary chambers. Providing the reagent in the secondary chambers has the advantage that it permits different reagents and therefore different assays in each of the secondary chambers.

For an *E. coli* most probable number assay the reagent is preferably a cleavable indicator such as 4-methylumbellifery-beta-D-glucuronide (MUG) or 5-bromo-4-chhloro-3-indolyl-beta-D-glucuronide (X-gluc), or a related or derivatized compound, which produces a fluorescent or chromogenic signal on cleavage by enzymes specific to *E. coli* bacteria.

In some embodiments it is preferred to carry out a dual assay wherein two assays are carried out in the same secondary chamber and each assay provides a different signal to the other such that the two signals can be distinguished from each other. An example of a dual assay would be carrying out a colorimetric X-gal assay for total coliforms and a fluorescent MUG assay for beta-glucuronidase positive *E. Coli* cells. In other embodiments a triple or quadruple assay may be carried out.

Other reagents which may be used for various assays are those which produce a change in sample fluorescence, electromagnetic absorption, emission or transmission in either the visible or non-visible part of the electromagnetic spectrum. For a pH assay a colour change indicator may be suitable. For a cyanide assay a colour change reagent based on the isonicotinic/barbituric acid method may be suitable.

### Incubation

Once the assay reagent has been allowed to come into contact with the sample, it may be necessary to incubate the device under specific conditions (such as temperature) for a predetermined length of time. The device may be provided with features (for example notches, detents or tags) to allow it to be held securely in an incubation apparatus or stand during this time. It is important that the device is not disturbed during the incubation to avoid cross contamination resulting from liquid in the primary chamber splashing into the secondary chambers.

### Readout of results

Depending on the type of assay, the results may be read out through the external wall of the device adjacent to the secondary chambers (and when present the 'snorkel') by eye, by camera or by an alternative suitable photometric apparatus. The device may optionally be configured to fit into and be held securely in such apparatus, which may optionally be the same apparatus as the incubation apparatus.

According to certain embodiments, the results may be read out by a micro sensor device, for example a printed electronic sensor, which is provided as part of the test device positioned in or as part of the wall of a secondary chamber.

### Methods of use

According to a second aspect of the invention there is provided a method of carrying out a most probable number assay for a microbial organism, comprising the steps of:
A, introducing a liquid sample (40) into a primary chamber (10) of a test device (1),
B, positioning the test device (1) such that an aliquot of the liquid sample from the primary chamber (10) flows into each of a multiplicity of secondary chambers (21, 22, 23, 24, 25, 26),
C, inverting the test device such that the aliquots of liquid sample in the secondary chambers (21, 22, 23, 24, 25, 26) are no longer in contact with the liquid sample remaining in the primary chamber (10),
D, allowing an assay reaction to take place in situ in the secondary chambers (21, 22, 23, 24, 25, 26) and, optionally the primary chamber (10),
E, reading the results of the assay reaction characterised in that the test device (1) is provided with a drip management system (80, 81, 82, 83, 84), wherein the test device is arranged so that, when the device is in the second position, any drips and surface flows of liquid flowing out of the secondary chambers (21, 22, 23, 24, 25, 26) are encouraged to flow or drip downwards into, or at least towards the primary chamber (10) in order to move them away from the regions of the device comprising the secondary chambers (21, 22, 23, 24, 25, 26) where they might contribute to contamination occurring between secondary chambers (21, 22, 23, 24, 25, 26) and wherein the volume of the primary chamber (10) is at least an order of magnitude greater than the volume of each of the secondary chambers (21, 22, 23, 24, 25, 26).

The secondary chambers according to this second aspect may be as defined further with respect to the first aspect. For example, the secondary chambers may optionally contain absorbent material as described above.

According to certain embodiments of the second aspect of the invention the various features described above with reference to the first aspect of the invention, for example features specifying chamber volumes, number and configuration, the presence of an optional snorkel, materials of construction, the form of the absorbent material, the liquid sample identity, the assay or assays carried out, the reagents used, the drip management system, the contamination management system and the reading of the results are all applicable to methods of the invention. The reverse also applies.

According to certain embodiments the test device consists of a lid portion and a base portion and step A is carried out by pouring the liquid sample into the base portions or scooping up the liquid sample into the base portions and screwing on the lid portion. Optionally, a plain lid may be temporarily screwed onto the base portion after the liquid sample has been taken into the base portion but before the lid portion comprising the secondary chambers has been attached to the base portion. The use of a temporary plain lid may be useful in ensuring secure transport of the sample.

Preferably, the secondary chambers are provided in the lid portion. Accordingly, step B may be carried out by inverting the device so that the secondary chambers held in the lid are below the primary chamber. In step C the device is inverted again so that the lid, holding the secondary chambers, is uppermost. Preferably, aliquots of liquid sample are maintained in the secondary chambers by the presence of absorbent material as described above in reference to the first aspect of the invention.

According to certain embodiments of the invention there is provided a method wherein step A is carried out by pouring the liquid sample into the base portion or scooping up the liquid sample into the base portion and screwing on the lid portion. The method then proceeds to step E and is followed by the steps of replacing the lid with a further lid in order to carry out a different or duplicate assay in accordance with steps B to E.

In preferred embodiments, the liquid sample is a water sample and the assay comprises a most probable number assay for coliform bacteria such as *E. coli.* Accordingly, there is an additional step between step A and B or step B and C of mixing the sample in the primary chamber with a fluorescent or colorimetric reagent suitable for the growth and detection of coliform bacteria (for example a reagent specific for *E. coli*). The device can then be inverted in step C and step D will involve an incubation step under conditions suitable for the proliferation of *E. coli* in those chambers in which such bacterial cells are present. Typically, such an incubation involves an incubation at approximately 37°C (for example from 32 to 40°C or from 34 to 39°C) for at least 12, 14, 16, 18, 20 or 24 hours.

In step E the results of the assay are read, which is a process essentially involving the counting of the number of secondary chambers which are positive and negative for the growth of coliform cells (for example *E. coli* cells).

According to certain preferred embodiments, methods of the invention involve use of a device in accordance with the invention according to the various device embodiments disclosed herein.

### Decontamination

The contents of the test device may, in accordance with some uses, be toxic after use. For example, they may contain hazardous chemicals or microbes. Advantageously, the device does not need to be opened after use and so the hazardous content is retained safely until appropriate disposal.

According to certain embodiments of the method of the invention, there is an additional optional step, step F, following step E, in which the test device is decontaminated either by adding a microbicide compound to the liquid in the device or by heating the device to a temperature sufficient to kill harmful organisms. The latter feature has the advantage that the organisms can be killed without the device needing to be opened. Heating may also advantageously kill harmful organisms on the external surface of the device and/or on surfaces of the incubator.

### Apparatus

There is also disclosed an assay apparatus comprising a combination of a device of the invention and a means to incubate a liquid sample held in the device for a period of time at a predetermined temperature.

The incubation means may, in certain embodiments, be an electrical heater. The heater may be arranged to maintain the test device at a temperature and for a time period suitable for microbial growth of the organism of interest.

Optionally, the apparatus may further comprise a means to detect a photometric signal from the test assay. For example, the apparatus may comprise a camera or a photometer or a fluorimeter or a device for detecting photon absorption. Alternatively, the device may comprise means for receiving data from sensors inside the secondary chambers.

Optionally, the apparatus may be arranged so that the incubation means is also able to heat the test device and its content to a temperature sufficient to kill harmful microbes which may be inside the device prior to safe disposal of the device. Harmful organisms on the external surface of the device and/or surfaces of the incubator are also advantageously killed.

The invention will now be illustrated by reference to the following non-limiting examples.

### Example 1. - Example user instructions for use of the device of the invention in a Most Probable Number method for quantifying Total Coliforms and Escherichia coli (E.coli) in crop irrigation.

### 1. Principle

A sample pot is filled with 100 ml of water. It is then taken to an indoor environment with an electricity supply where the test is performed. A "snap pack" of powdered EC Blue 100P ™ growth medium as supplied by Nissui Pharmaceutical Co. Ltd. (Tokyo) is added to the sample pot and, after shaking, the original sample pot lid is replaced with a diagnostic lid in accordance with the invention. The diagnostic lid is as illustrated in figure 10 and has 10 secondary chambers filled with absorbent material, 8 of which have a volume of 0.2 ml and 2 of which have a volume of 2 ml as well as a central snorkel chamber. The device is inverted once, returned to a position with the lid uppermost and then placed in a programmable heating block where the device is held at a constant temperature for 24 hours. The numbers of positive growth chambers on the diagnostic lid are counted under white light (blue-green chambers) and again under UV light (fluorescent blue). The estimated numbers of Total Coliforms (blue-green chambers) and *E.coli* (fluorescent blue) are read from a Most Probable Number (MPN) table (see below).

### 2. Test Procedure

NB During sample collection and processing there is a need for clean hands and contact with the inner surfaces of the device should be avoided. Failure to observe this may increase the risk of cross contamination.

### 2.1. Sample Collection

Filling could be, for example, from a tap or by dipping the pot into the water source. Remove the sample pot lid from the sample pot and fill to the 100 ml mark; replace the sample pot lid. The inside of the sample pot lid or sample pot should not come into contact with fingers or other non-sterile surfaces.

The sample should be taken (within 1 hour) to the indoor environment where it is to be tested and not left in direct sunlight or allowed to freeze.

Samples should be labelled with the sample reference, time, date, source and type of sample.

Note: If required further generic information on water sampling may be found at https://www.gov.uk/government/uploads/system/uploads/attachment_data/file/316769/MoDW-2-232.pdf

### 2.2 Addition of growth medium

The following is best performed indoors on a clean work surface.

Unscrew the sample pot lid and place it on a clean surface with the inside of the lid facing upwards. As above ensure that the inside of the lid or pot is not contaminated e.g. by touching with fingers.

Pour away any excess water so that 100 ml remains in the sample pot.

Open the EC Blue 100P™ growth medium snap pack according to the three diagrams in the manufacturer's instructions and pour into the sample pot.

Replace the sample pot lid and shake for a few seconds to start to dissolve the growth medium. Leave for two minutes, shaking several times during this time period to dissolve completely.

Remove the sample pot lid once more and replace with a diagnostic lid (taking precautions as above) ensuring that it is on tightly, but do not overtighten.

Invert the device with the diagnostic lid in place and leave for one minute, tapping twice to allow any air bubbles to escape from the surfaces of the growth chambers. Return the device to an upright position and tap twice to let any drips fall into the main chamber.

### 2.3 Incubation

Turn on the programmable heating block. This should show a target temperature of 37 degrees in the lower section of screen, with the rising block temperature in the upper section.

Remove the black heating block cover and the glass shield. Place the device with the diagnostic lid uppermost in the heating block, fitting the lug in the diagnostic lid into the notch in the heating block. Replace both the glass shield and the black cover. Press the start button. The programme will run for 24 hours.

Do not remove the device at any point during this process. Please note that shaking or inversion of the device during the incubation period will invalidate the result.

When the programme has finished the result must be read within 4 hours.

### 2.4. Reading and interpretation of results

For Total Coliforms: Press the rear button on the black cover to illuminate the top of the diagnostic lid with white light. Do not remove the device from the block.

Count the number of positive (blue-green) growth chambers (out of a possible 11 [- for the purposes of user instructions the central snorkel chamber of counted as a "growth chamber" together with the 10 secondary chambers] and mark the positive chambers on the left hand diagram on the Data Recording Sheet.

For *E*. *coli*: Press the front button on the black cover to illuminate the top of the diagnostic lid with UV light. Do not remove the device from the block.

Count the number of positive (fluorescent blue) growth chambers (out of a possible 11) and mark the positive chambers on the right hand diagram on the Data Recording Sheet.

Once the numbers of positive growth chambers have been ascertained, refer to the supplied Most Probable Number table (below). The Most Probable Number results are read from the table.

### 2.5 Disposal

Do not open the device or pour the contents into the waste.

After use, the intact device (sample pot and diagnostic lid), the standard sample pot lid and the snap pack, should be placed in the microbiological waste container provided and the lid secured.

### 3. Limitations of Method

This method is intended to monitor trends and approximate levels of contamination in raw water - e.g. that used for irrigation of food crops - with typical counts up to 1000 *E.coli* per 100 ml.

The results are estimates with an associated confidence interval for each result (not shown on the MPN table).

Note: if required, further information on the MPN method and confidence intervals is on the World Health Organization website: http://www.who.int/water_sanitation_health/dwq/2edvol3i.pdf

EC Blue 100P™ Manufacturer's instructions may be downloaded from http://www.medica-tec.com/arg/files/NISSUI%20-%20Inserto%20EC%20Blue.pdf

**MPN table**

| Total number of positive chambers | Most Probable Number (cfu/100 ml) |
|---|---|
| 0 | 0 |
| 1 | 3 |
| 2 | 19 |
| 3 | 48 |
| 4 | 89 |
| 5 | 150 |
| 6 | 230 |
| 7 | 330 |
| 8 | 470 |
| 9 | 660 |
| 10 | 990 |
| 11 | Not defined |

### Example 2. Comparison with current (prior art) device

### Introduction

A direct comparison between Brightwater device in accordance with the invention and the commonly used existing IDEXX® Quantitray 2000™ device (utilising Colilert-18™ reagent) was carried out using spiked samples of water containing *Escherichia coli.* The Brightwater (BW) devices utilise a Most Probable Number (MPN) system in the form of a diagnostic lid incorporating 3 large chambers (the snorkel/primary chamber having a 94.4 ml volume and 2x 2 ml secondary chambers) and 8 small chambers (8 secondary chambers of 0.2 ml each) for determining target organisms in 100 ml of sample. Of the 3 large chambers, 1 is in constant contact with the test sample through the use of a longer tube (snorkel).

The IDEXX® Quantitray 2000 system also utilises a MPN system for enumerating target organisms in 100 ml samples. The system incorporates 49 large 'wells' and 48 smaller 'wells'. Both the BW units and the IDEXX® system utilised Coliert-18 (IDEXX®) as the reagent, utilising fluorescence to signify a positive result. The inoculum was determined by the pour plate method to give accurate levels of target numbers.

The experiment was repeated on 2 separate occasions using a total of 24 Brightwater devices and 8 Quantitray 2000 devices.

Target ranges of *E*. *coli* varied from 500 cfu per 100 ml in the first set of experiments to 150 cfu per 100 ml in the second set of experiments. This was felt to be a realistic target range based on the anticipated use and water matrix being considered for testing.

### Aims:

1. Compare MPN values of BW device with Quantitray 2000 using Colilert-18 reagent.
2. Show there is no significant difference between the results from both devices using statistical analysis of the data.

### Method

An overnight culture of *E*. *coli* (NCTC 9001, Public Health England) was grown up in Nutrient Broth (Oxoid) and serially diluted in Maximum Recovery Diluent (Oxoid) to give low level colony forming units (cfu) per millilitre. In order to accurately extrapolate the inoculum of the broth, a 100µl aliquot of the 10-6 dilution of *E*. *coli* was spread on duplicate plates of Tryptone Soya Agar (Oxoid) and counted.

### -Setting up the spiked water sample

Two litres of sterile distilled water were inoculated with a pure culture of *E*. *coli* giving a theoretical level of 150 and 500 cfu per 100 ml.

### -Setting up test items (BW devices and Quantitray 2000's)

### -Brightwater devices

A total of 12 BW devices were available for each experiment. Using a UKAS calibrated balance (SciChem), 100 ml of spiked water was weighed into each of the BW units. To each unit containing 100 ml of sample was added a single sachet of Coliert-18 reagent (IDEXX®). The standard sample pot lid was replaced and the reagent was left undisturbed for 5 minutes.

After 5 minutes the sample containers were gently inverted 3 times to ensure the reagent had completely dissolved.

Once completely dissolved, the standard lid of each unit was replaced with the diagnostic lid.

The units were then inverted to ensure all chambers became completely submerged in the spiked water sample. After 1 minute the unit was tapped firmly 3 times to remove any air bubbles.

The units were then re-inverted and incubated at 37°C ± 0.5°C for 18 hours.

### -Quantitray 2000's

Using a UKAS calibrated balance (SciChem), 100 ml of spiked water was weighed into each of four 120 ml vessels (IDEXX®). To each vessel containing 100 ml of sample was added a single sachet of Colilert-18 reagent. The lid was replaced and the reagent was left undisturbed for 5 minutes. After 5 minutes the vessels were gently inverted 3 times to ensure the reagent had completely dissolved. Each sample was then transferred to a Quantitray 2000 and sealed in the IDEXX® Quantitray Sealer. The Quantitrays were incubated at 37°C ± 0.5°C for 18 hours.

### -Reading results from the 2 experiments

For the Brightwater units and the IDEXX ® Quantitray 2000's a positive reaction was deemed as a chamber showing fluorescence under a UV lamp at 365 nm wavelength. The number of positive chambers was then noted and the appropriate MPN table (as used in Example 1) used to determine the Most Probable Number of the sample.

### Results

### -Brightwater units

Fluorescence was easily visualised through the lids of the BW devices under UV light (365nm wavelength) and the number of positive chambers out of a possible maximum of 11 were recorded.

### -Brightwater units Data set 1 (inoculum of approx. 500 cfu per 100 ml)

| BW unit ref No | No of +ve large chambers * | No of +ve small chambers * | Total number of +ve chambers * | MPN from look-up table | 95% CI Lower | 95% CI lower |
|---|---|---|---|---|---|---|
| 1 | 3 | 5 | 8 | 470 | 170 | 1050 |
| 2 | 3 | 5 | 8 | 470 | 170 | 1050 |
| 3 | 3 | 6 | 9 | 660 | 220 | 1460 |
| 4 | 3 | 6 | 9 | 660 | 220 | 1460 |
| 5 | 3 | 6 | 9 | 660 | 220 | 1460 |
| 6 | 3 | 6 | 9 | 660 | 220 | 1460 |
| 7 | 3 | 5 | 8 | 470 | 170 | 1050 |
| 8 | 3 | 5 | 8 | 470 | 170 | 1050 |
| 9 | 3 | 4 | 7 | 330 | 110 | 780 |
| 10 | 3 | 6 | 9 | 660 | 220 | 1460 |
| 11 | 3 | 6 | 9 | 660 | 220 | 1460 |
| 12 | 3 | 5 | 8 | 470 | 170 | 1050 |
| | | | | Mean MPN = 553 | | |

### -Brightwater units Data set 2 (inoculum of approx. 150 cfu per 100 ml)

| BW unit ref No | No of +ve large chambers * | No of +ve small chambers * | Total number of +ve chambers * | MPN from look-up table | 95% CI Lower | 95% CI lower |
|---|---|---|---|---|---|---|
| 1 | 3 | 3 | 6 | 230 | 58 | 590 |
| 2 | 3 | 2 | 5 | 150 | 39 | 490 |
| 3 | 3 | 4 | 7 | 330 | 110 | 780 |
| 4 | 3 | 3 | 6 | 230 | 58 | 590 |
| 5 | 3 | 4 | 7 | 330 | 110 | 780 |
| 6 | 3 | 2 | 5 | 150 | 39 | 490 |
| 7 | 3 | 3 | 6 | 230 | 58 | 590 |
| 8 | 3 | 0 | 3 | 48 | 7 | 210 |
| 9 | 3 | 2 | 5 | 150 | 39 | 490 |
| 10 | 3 | 2 | 5 | 150 | 39 | 490 |
| 11 | 3 | 2 | 5 | 150 | 39 | 490 |
| 12 | 3 | 3 | 6 | 230 | 58 | 590 |
| | | | | Mean MPN = 198 | | |

| | | | | | | |
|---|---|---|---|---|---|---|
| *N. B. for the purposes of the examples and from a user perspective both the primary chamber and the secondary chambers are regarded simply as "chambers" to be counted. The primary assay chamber being assessed by means of the snorkel which is mounted on the diagnostic lid of the device and appears as a large chamber. | | | | | | |

### -Quantitray 2000 Data set 1 (inoculum of approx. 500 cfu per 100 ml)

| Quanti-tray ref | No of +ve large chambers | No of +ve small chambers | MPN from look-up table | 95% CI lower | 95% CI upper |
|---|---|---|---|---|---|
| 1 | 49 | 22 | 387 | 246 | 567 |
| 2 | 49 | 23 | 411 | 261 | 619 |
| 3 | 49 | 27 | 517 | 338 | 764 |
| 4 | 49 | 24 | 435 | 276 | 650 |
| | | | Mean MPN= 438 | | |

### -Quantitray 2000 Data set 2 (inoculum of approx. 150 cfu per 100 ml)

| Quanti-tray ref | No of +ve large chambers | No of +ve small chambers | MPN from look-up table | 95% CI lower | 95% CI upper |
|---|---|---|---|---|---|
| 1 | 45 | 12 | 144 | 103 | 195 |
| 2 | 47 | 11 | 166 | 115 | 234 |
| 3 | 48 | 14 | 210 | 146 | 301 |
| 4 | 47 | 14 | 161 | 111 | 226 |
| | | | Mean MPN= 170 | | |

### Data analysis

2-way analysis of variance was carried out on the data. That analysis concluded that there is no significant evidence of a difference between the two methods (P=0.301).

### Conclusions

The data generated from the experiments does not show that there is a significant difference in results between the two testing systems. Visualisation of positive chambers was very good in both systems under UV light at 365 nm. The aim of the experiments to demonstrate that the methods of the invention yield comparable results to those of the prior art was been achieved.

### References

1. The Microbiology of Drinking Water (2009) - Part 4 - Methods for the isolation and enumeration of coliform bacteria and Escherichia coli (including E. coli O157:H7). Environment Agency (UK)

## Claims

1. A test device (1) for carrying out a most probable number microbial assay comprising a primary chamber (10) to receive an initial liquid sample (40) and a multiplicity of secondary chambers (21, 22, 23, 24, 25, 26) arranged to each receive an aliquot of the initial liquid sample from the primary chamber, wherein the test device is movable in use between two positions, a first position and a second position; **characterised in that** in the first position the secondary chambers (21, 22, 23, 24, 25, 26 are situated below the primary chamber (10) such that an aliquot of the initial liquid sample (40) flows under gravity from the primary chamber (10) to each of the secondary chambers (21, 22, 23, 24, 25, 26); and that in the second position the secondary chambers (21, 22, 23, 24, 25, 26) are located above the primary chamber (10) such that the liquid in the primary chamber (10) is not able to flow under gravity from the primary chamber (10) to each of the secondary chambers (21, 22, 23, 24, 25, 26); the aliquots of liquid sample which previously flowed under gravity from the primary chamber (10) to the secondary chambers (21, 22, 23, 24, 25, 26) when the device was in the first position being retained in the secondary chambers (21, 22, 23, 24, 25, 26) against the force of gravity and wherein the device is arranged so that an assay may be carried out *in situ* in at least some of the secondary chambers (21, 22, 23, 24, 25, 26), **characterised in that** the test device (1) is provided with a drip management system (80, 81, 82, 83, 84), wherein the test device is arranged so that, when the device is in the second position, any drips and surface flows of liquid flowing out of the secondary chambers (21, 22, 23, 24, 25, 26) are encouraged to flow or drip downwards into, or at least towards the primary chamber (10) in order to move them away from the regions of the device comprising the secondary chambers (21, 22, 23, 24, 25, 26) where they might contribute to contamination occurring between secondary chambers (21, 22, 23, 24, 25, 26) and wherein the volume of the primary chamber (10) is at least an order of magnitude greater than the volume of each of the secondary chambers (21, 22, 23, 24, 25, 26).

2. A test device according to claim 1, wherein the secondary chambers comprise absorbent material to retain the liquid in the secondary chambers.

3. A test device according to claim 2, wherein the absorbent material comprises microstructures and liquid trapping and retaining surfaces moulded into the walls of the secondary chamber.

4. A test device according to claim 3, wherein the absorbent material microstructures are capillary structures, arranged to abstract, retain and/or contain a portion of liquid.

5. A test device according to claims 1-4, wherein the device comprises a base portion and a lid portion which screw together to form the complete device.

6. A test device according to claim 5, wherein the secondary chambers are mounted in the lid portion.

7. A test device according to claim 2, 3, 4, 5 or 6, wherein the lid portion additionally comprises a chamber containing absorbent material, which reaches into the primary chamber such that it is in liquid communication with the initial liquid sample in the primary chamber when the test device is in either the first or second position.

8. A method of carrying out a most probable number assay for a microbial organism, comprising the steps of:
A, introducing a liquid sample (40) into a primary chamber (10) of a test device (1),
B, positioning the test device (1) such that an aliquot of the liquid sample from the primary chamber (10) flows into each of a multiplicity of secondary chambers (21, 22, 23, 24, 25, 26),
C, inverting the test device such that the aliquots of liquid sample in the secondary chambers (21, 22, 23, 24, 25, 26) are no longer in contact with the liquid sample remaining in the primary chamber (10),
D, allowing an assay reaction to take place *in situ* in the secondary chambers (21, 22, 23, 24, 25, 26) and, optionally the primary chamber (10),
E, reading the results of the assay reaction **characterised in that** the test device (1) is provided with a drip management system (80, 81, 82, 83, 84), wherein the test device is arranged so that, when the device is in the second position, any drips and surface flows of liquid flowing out of the secondary chambers (21, 22, 23, 24, 25, 26) are encouraged to flow or drip downwards into, or at least towards the primary chamber (10) in order to move them away from the regions of the device comprising the secondary chambers (21, 22, 23, 24, 25, 26) where they might contribute to contamination occurring between secondary chambers (21, 22, 23, 24, 25, 26) and wherein the volume of the primary chamber (10) is at least an order of magnitude greater than the volume of each of the secondary chambers (21, 22, 23, 24, 25, 26).

9. A method according to claim 8, wherein the liquid sample is a water sample.

10. A method according to claim 8, wherein the liquid sample is a urine sample.

11. A method according to claim 8, wherein the microbial organism is a coliform bacteria, for example *E. coli.*

12. A method according to claim 11 wherein the assay is a dual assay wherein two assays are carried out per secondary region, the first assay being for total coliforms and the second assay being for beta-glucuronidase positive *E. Coli.*

13. A method according to claim 11 or 12, wherein step D comprises an incubation at from 32 to 44°C (for example from 32 to 40 °C) for sufficient time to allow the bacteria to multiply to detectable levels.

14. A method according to claim 8, 9, 10, 11, 12 or 13, comprising a further step F, following step E :-
F, heating the device and its content to a temperature and for a time sufficient to kill harmful microbes which may be in the device.

15. A method according to any of claims 8, 9, or 10, wherein the assay is an assay for a chemical compound.

16. A method according to any of claims 8 to 15, wherein the test device is as defined in any of claims 1 to 7.

17. A test device according to any of claims 1 to 7, wherein the device is arranged so that colour change test reactions taking place in the secondary chambers may be captured using a camera and image recognition software, for example as part of a 2D barcode.

## Patentansprüche

1. Testvorrichtung (1) zum Durchführen eines Most-Probable-Number-Tests auf Mikroorganismen, umfassend eine Primärkammer (10) zur Aufnahme einer initialen Flüssigkeitsprobe (40) und eine Mehrzahl von Sekundärkammern (21, 22, 23, 24, 25, 26), die so gestaltet sind, dass jede davon ein Aliquot der initialen Flüssigkeitsprobe aus der Primärkammer erhält, wobei die Testvorrichtung bei Gebrauch zwischen zwei Positionen beweglich ist, einer ersten Position und einer zweiten Position; **dadurch gekennzeichnet, dass** in der ersten Position die Sekundärkammern (21, 22, 23, 24, 25, 26) sich unterhalb der Primärkammer (10) befinden, so dass ein Aliquot der initialen Flüssigkeitsprobe (40) unter dem Einfluss der Schwerkraft von der Primärkammer (10) in jede der Sekundärkammern (21, 22, 23, 24, 25, 26) fließt; und dass in der zweiten Position die Sekundärkammern (21, 22, 23, 24, 25, 26) sich oberhalb der Primärkammer (10) befinden, so dass die Flüssigkeit in der Primärkammer (10) nicht unter dem Einfluss der Schwerkraft von der Primärkammer (10) in jede der Sekundärkammern (21, 22, 23, 24, 25, 26) fließen kann; wobei die Aliquote der Flüssigkeitsprobe, die zuvor unter dem Einfluss der Schwerkraft von der Primärkammer (10) in die Sekundärkammern (21, 22, 23, 24, 25, 26) flossen, als die Vorrichtung sich in der ersten Position befand, in den Sekundärkammern (21, 22, 23, 24, 25, 26) gegen die Schwerkraft gehalten werden, und wobei die Vorrichtung so gestaltet ist, dass ein Test in wenigstens einigen der Sekundärkammern (21, 22, 23, 24, 25, 26) *in situ* durchgeführt werden kann, **dadurch gekennzeichnet, dass** die Testvorrichtung (1) mit einem Tropfmanagementsystem (80, 81, 82, 83, 84) ausgestattet ist, wobei die Testvorrichtung so gestaltet ist, dass wenn die Vorrichtung sich in der zweiten Position befindet, jegliche Tropfen und Oberflächenströme der Flüssigkeit, die aus den Sekundärkammern (21, 22, 23, 24, 25, 26) fließen, animiert werden, nach unten in die oder wenigstens in Richtung der Primärkammer (10) zu fließen oder tropfen, um sie von den Bereichen der Vorrichtung weg zu bewegen, die die Sekundärkammern (21, 22, 23, 24, 25, 26) umfassen, wo sie zu einer zwischen Sekundärkammern (21, 22, 23, 24, 25, 26) stattfindenden Kontaminierung beitragen könnten, und wobei das Volumen der Primärkammer (10) wenigstens eine Größenordnung größer ist als das Volumen einer jeden der Sekundärkammern (21, 22, 23, 24, 25, 26).

2. Testvorrichtung gemäß Anspruch 1, wobei die Sekundärkammern absorbierendes Material umfassen, um die Flüssigkeit in den Sekundärkammern zu halten.

3. Testvorrichtung gemäß Anspruch 2, wobei das absorbierende Material Mikrostrukturen und flüssigkeitseinfangende und -zurückhaltende Oberflächen, die in die Wände der Sekundärkammern eingeformt wurden, umfasst.

4. Testvorrichtung gemäß Anspruch 3, wobei die Mikrostrukturen des absorbierenden Materials Kapillarstrukturen sind, die so gestaltet sind, dass sie einen Teil der Flüssigkeit abstrahieren, zurückhalten und/oder enthalten.

5. Testvorrichtung gemäß den Ansprüchen 1-4, wobei die Vorrichtung einen Basisteil und einen Deckelteil umfasst, welche sich zusammenschrauben lassen, um die vollständige Vorrichtung zu bilden.

6. Testvorrichtung gemäß Anspruch 5, wobei die Sekundärkammern in dem Deckelteil montiert sind.

7. Testvorrichtung gemäß Anspruch 2, 3, 4, 5 oder 6, wobei der Deckelteil zusätzlich eine Kammer umfasst, die absorbierendes Material enthält, welches in die Primärkammer reicht, so dass es in flüssiger Kommunikation mit der initialen Flüssigkeitsprobe in der Primärkammer steht, wenn sich die Testvorrichtung entweder in der ersten oder in der zweiten Position befindet.

8. Verfahren zur Durchführung eines Most-Probable-Number-Tests auf einen Mikroorganismus, umfassend die Schritte:
A, Einbringen einer Flüssigkeitsprobe (40) in eine Primärkammer (10) einer Testvorrichtung (1),
B, Positionieren der Testvorrichtung (1), so dass ein Aliquot der Flüssigkeitsprobe aus der Primärkammer (10) in jede von einer Mehrzahl von Sekundärkammern (21, 22, 23, 24, 25, 26) fließt,
C, Invertieren der Testvorrichtung, so dass die Aliquote der Flüssigkeitsprobe in den Sekundärkammern (21, 22, 23, 24, 25, 26) nicht länger mit der in der Primärkammer (10) verbliebenen Flüssigkeitsprobe in Kontakt stehen,
D, Ablaufenlassen einer Testreaktion *in situ* in den Sekundärkammern (21, 22, 23, 24, 25, 26) und gegebenenfalls der Primärkammer (10),
E, Ablesen der Ergebnisse der Testreaktion, **dadurch gekennzeichnet, dass** die Testvorrichtung (1) mit einem Tropfmanagementsystem (80, 81, 82, 83, 84) ausgestattet ist, wobei die Testvorrichtung so gestaltet ist, dass wenn die Vorrichtung sich in der zweiten Position befindet, jegliche Tropfen und Oberflächenströme der Flüssigkeit, die aus den Sekundärkammern (21, 22, 23, 24, 25, 26) fließen, animiert werden, nach unten in die oder wenigstens in Richtung der Primärkammer (10) zu fließen oder tropfen, um sie von den Bereichen der Vorrichtung weg zu bewegen, die die Sekundärkammern (21, 22, 23, 24, 25, 26) umfassen, wo sie zu einer zwischen Sekundärkammern (21, 22, 23, 24, 25, 26) stattfindenden Kontaminierung beitragen könnten, und wobei das Volumen der Primärkammer (10) wenigstens eine Größenordnung größer ist als das Volumen einer jeden der Sekundärkammern (21, 22, 23, 24, 25, 26).

9. Verfahren gemäß Anspruch 8, wobei die Flüssigkeitsprobe eine Wasserprobe ist.

10. Verfahren gemäß Anspruch 8, wobei die Flüssigkeitsprobe eine Urinprobe ist.

11. Verfahren gemäß Anspruch 8, wobei der Mikroorganismus coliforme Bakterien ist, zum Beispiel *E. coli.*

12. Verfahren gemäß Anspruch 1, wobei der Test ein zweifacher Test ist, bei dem zwei Tests pro Sekundärbereich durchgeführt werden, wobei der erste Test die Gesamtcoliforme bestimmt und der zweite Test beta-Glucuronidase-positive *E. coli* bestimmt.

13. Verfahren gemäß Anspruch 11 oder 12, wobei Schritt D eine Inkubation bei 32 bis 44 °C (zum Beispiel 32 bis 40 °C) für eine ausreichende Zeit, um das Vermehren der Bakterien auf nachweisbare Levels zu ermöglichen, umfasst.

14. Verfahren gemäß Anspruch 8, 9, 10, 11, 12 oder 13, das einen weiteren Schritt F, der auf Schritt E folgt, umfasst:
F, Erwärmen der Vorrichtung und deren Inhalte auf eine Temperatur und für eine ausreichende Zeit, um schädliche Mikroben abzutöten, die sich in der Vorrichtung befinden können.

15. Verfahren gemäß einem der Ansprüche 8, 9 oder 10, wobei der Test ein Test für eine chemische Verbindung ist.

16. Verfahren gemäß einem der Ansprüche 8 bis 15, wobei die Testvorrichtung wie in einem der Ansprüche 1 bis 7 definiert ist.

17. Testvorrichtung gemäß einem der Ansprüche 1 bis 7, wobei die Vorrichtung so gestaltet ist, dass Farbveränderungstestreaktionen, die in den Sekundärkammern stattfinden, mittels einer Kamera und einer Bilderkennungssoftware zum Beispiel als Teil eines 2D-Barcodes eingefangen werden können.

## Revendications

1. Dispositif d'essai (1) pour réaliser un essai de dénombrement microbien comprenant une chambre principale (10) pour recevoir un échantillon de liquide initial (40) et plusieurs chambres secondaires (21, 22, 23, 24, 25, 26) configurées pour recevoir chacune une aliquote de l'échantillon de liquide initial de la chambre principale, dans lequel le dispositif d'essai peut être utilisé dans deux positions, une première position et une seconde position ; **caractérisées en ce que** dans la première position, les chambres secondaires (21, 22, 23, 24, 25, 26) sont situées en dessous la chambre principale (10) de sorte qu'une aliquote de l'échantillon de liquide initial (40) s'écoule sous l'effet de la gravité depuis la chambre principale (10) vers chacune des chambres secondaires (21, 22, 23, 24, 25, 26) ; et **en ce que** dans la seconde position, les chambres secondaires (21, 22, 23, 24, 25, 26) sont situées au-dessus de la chambre principale (10) de sorte que le liquide dans la chambre principale (10) ne peut pas s'écouler sous l'effet de la gravité depuis la chambre principale (10) vers chacune des chambres secondaires (21, 22, 23, 24, 25, 26) ; les aliquotes de l'échantillon de liquide qui s'écoulaient précédemment sous l'effet de la gravité depuis la chambre principale (10) vers les chambres secondaires (21, 22, 23, 24, 25, 26) lorsque le dispositif était dans la première position étant retenues dans les chambres secondaires (21, 22, 23, 24, 25, 26) contre la force de gravité et dans lequel le dispositif est configuré afin qu'un essai puisse être réalisé *in situ* dans au moins certaines des chambres secondaires (21, 22, 23, 24, 25, 26), **caractérisé en ce que** le dispositif d'essai (1) est équipé d'un système de gestion de gouttes (80, 81, 82, 83, 84), dans lequel le dispositif d'essai est configuré de sorte que, lorsque le dispositif se trouve dans la seconde position, les gouttes et l'écoulement de surface du liquide s'écoulant hors des chambres secondaires (21, 22, 23, 24, 25, 26) soient encouragés à s'écouler ou goutter vers le bas dans, ou au moins vers, la chambre principale (10) afin de les éloigner des régions du dispositif comprenant les chambres secondaires (21, 22, 23, 24, 25, 26) où elles pourraient contribuer à la contamination entre les chambres secondaires (21, 22, 23, 24, 25, 26) et dans lequel le volume de la chambre principale (10) est au moins d'un ordre de grandeur supérieur au volume de chacune des chambres secondaires (21, 22, 23, 24, 25, 26).

2. Dispositif d'essai selon la revendication 1, dans lequel les chambres secondaires comprennent un matériau absorbant pour retenir le liquide dans les chambres secondaires.

3. Dispositif d'essai selon la revendication 2, dans lequel le matériau absorbant comprend des microstructures, ainsi que des surfaces pour piéger et retenir le liquide, moulées dans les parois de la chambre secondaire.

4. Dispositif d'essai selon la revendication 3, dans lequel les microstructures du matériau absorbant sont des structures capillaires, configurées pour extraire, retenir et/ou contenir une partie du liquide.

5. Dispositif d'essai selon les revendications 1 à 4, dans lequel le dispositif comprend une partie de base et une partie de couvercle qui sont vissées ensemble pour former le dispositif complet.

6. Dispositif d'essai selon la revendication 5, dans lequel les chambres secondaires sont montées dans la partie de couvercle.

7. Dispositif d'essai selon les revendications 2, 3, 4, 5 ou 6, dans lequel la partie de couvercle comprend également une chambre contenant un matériau absorbant, qui réagit dans la chambre principale de sorte qu'il se trouve en communication liquide avec l'échantillon de liquide initial dans la chambre principale lorsque le dispositif d'essai est soit dans la première, soit dans la seconde position.

8. Méthode pour réaliser un essai de dénombrement pour un organisme microbien, comprenant les étapes suivantes :
A. Introduction d'un échantillon de liquide (40) dans une chambre principale (10) d'un dispositif d'essai (1)
B. Positionnement du dispositif d'essai (1) de sorte qu'une aliquote de l'échantillon de liquide de la chambre principale (10) s'écoule dans chacune des différentes chambres secondaires (21, 22, 23, 24, 25, 26)
C. Inversion du dispositif d'essai de sorte que les aliquotes de l'échantillon de liquide dans les chambres secondaires (21, 22, 23, 24, 25, 26) ne soient plus en contact avec l'échantillon de liquide restant dans la chambre principale (10).
D. Autorisation qu'une réaction d'essai se déroule *in situ* dans les chambres secondaires (21, 22, 23, 24, 25, 26) et, en option dans la chambre principale (10)
E. Relevé des résultats de la réaction d'essai **caractérisé en ce que** le dispositif d'essai (1) est équipé d'un système de gestion de gouttes (80, 81, 82, 83, 84), dans lequel le dispositif d'essai est configuré de sorte que, lorsque le dispositif est dans la seconde position, les gouttes et l'écoulement de surface du liquide s'écoulant hors des chambres secondaires (21, 22, 23, 24, 25, 26) soient encouragés à s'écouler ou goutter vers le bas dans, ou au moins vers, la chambre principale (10) afin de les éloigner des régions du dispositif comprenant les chambres secondaires (21, 22, 23, 24, 25, 26) où elles pourraient contribuer à la contamination entre les chambres secondaires (21, 22, 23, 24, 25, 26) et dans lequel le volume de la chambre principale (10) est au moins d'un ordre de grandeur supérieur au volume de chacune des chambres secondaires (21, 22, 23, 24, 25, 26).

9. Méthode selon la revendication 8, dans laquelle l'échantillon de liquide est un échantillon d'eau.

10. Méthode selon la revendication 8, dans laquelle l'échantillon de liquide est un échantillon d'urine.

11. Méthode selon la revendication 8, dans laquelle l'organisme microbien est une bactérie coliforme, par exemple *E. coli.*

12. Méthode selon la revendication 11, dans laquelle l'essai est un double essai dans lequel deux essais sont réalisés pour chaque région secondaire, le premier essai étant pour l'ensemble des coliformes et le second pour l'*E*. *Coli* beta-glucuronidase positive.

13. Méthode selon les revendications 11 ou 12, dans laquelle l'étape D comprend une incubation de 32 à 44 °C (par exemple, de 32 à 40 °C) pendant une durée suffisante pour permettre à la bactérie de se multiplier jusqu'à des niveaux détectables.

14. Méthode selon les revendications 8, 9, 10, 11, 12 ou 13, comprenant une étape F, suivant l'étape E :
F. Chauffage du dispositif et de son contenu à une température et pendant une durée suffisante pour tuer les microbes nocifs qui peuvent être présents dans le dispositif.

15. Méthode selon les revendications 8, 9 et 10, dans laquelle l'essai est un essai pour un composé chimique.

16. Méthode selon les revendications 8 à 15, dans laquelle le dispositif d'essai est tel que défini dans les revendications 1 à 7.

17. Dispositif d'essai selon l'une quelconque des revendications 1 à 7, dans lequel le dispositif est configuré de sorte que les réactions d'essai de changement de couleur intervenant dans les chambres secondaires puissent être capturées à l'aide d'une caméra et d'un logiciel de reconnaissance d'image, par exemple, un code-barres 2D.
